(19)

Europäisches
Patentamt
European
Patent Office
Office européen
des brevets

(11) **EP 3 598 129 A1**

(12) **EUROPEAN PATENT APPLICATION**
published in accordance with Art. 153(4) EPC

(43) Date of publication:
**22.01.2020 Bulletin 2020/04**

(21) Application number: **18767630.9**

(22) Date of filing: **15.02.2018**

(51) Int Cl.:
*G01N 33/543* (2006.01)   *G01N 21/64* (2006.01)
*G01N 35/00* (2006.01)

(86) International application number:
**PCT/JP2018/005166**

(87) International publication number:
**WO 2018/168308 (20.09.2018 Gazette 2018/38)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD TN**

(30) Priority: **17.03.2017 JP 2017052777**

(71) Applicant: **Konica Minolta, Inc.
Tokyo 100-7015 (JP)**

(72) Inventors:
• **NODA, Tetsuya**
  **Tokyo 100-7015 (JP)**
• **IWASHITA, Atsuo**
  **Tokyo 100-7015 (JP)**
• **SHOUJI, Yuuya**
  **Tokyo 100-7015 (JP)**

(74) Representative: **Alton, Andrew
  Urquhart-Dykes & Lord LLP
  Arena Point
  Merrion Way
  Leeds LS2 8PA (GB)**

(54) **SAMPLE DETECTING SYSTEM**

(57)   To provide a sample detecting system capable of suppressing a temperature gradient and temperature unevenness in a sensor chip and controlling the temperature of a reaction portion with higher accuracy regardless of the temperature of an environment in which a sample detecting apparatus is disposed.

A sample detecting system includes a contact type temperature control unit disposed in contact with a sensor chip and a non-contact type temperature control unit disposed in non-contact with the sensor chip. The contact type temperature control unit includes a first temperature controller and a first temperature sensor that measures a temperature between the first temperature controller and the sensor chip, and performs feedback control of the first temperature controller on the basis of an output value of the first temperature sensor and a predetermined first temperature controller target temperature. The non-contact type temperature control unit includes a second temperature controller and a second temperature sensor that measures a temperature between the second temperature controller and the sensor chip, and performs feedback control of the second temperature controller on the basis of an output value of the second temperature sensor and a predetermined second temperature controller target temperature.

FIG. 1

EP 3 598 129 A1

**Description**

Technical Field

The present invention relates to a sample detecting system that detects a measurement target substance contained in a sensor chip, and more specifically to a sample detecting system capable of strictly controlling the temperature of a reaction portion of the sensor chip on which the measurement target substance is immobilized.

Background Art

Conventionally, in a case of detecting an extremely minute substance, various sample detecting methods capable of detecting such a substance by applying a physical phenomenon of the substance have been proposed.

As such a sample detecting method, for example, immunoassay for measuring presence or absence of a measurement target substance and the amount thereof utilizing an antigen-antibody reaction between an antigen that is a measurement target substance contained in a sample liquid and an antibody or an antigen labeled with a labeling substance is known.

Examples of immunoassay include enzyme immunoassay (EIA) using an enzyme as a labeling substance and fluorescence immunoassay (FIA) using a fluorescent substance as a labeling substance.

Examples of a sample detecting apparatus utilizing fluorescence immunoassay include a surface plasmon resonance apparatus (hereinafter, also referred to as "SPR apparatus") that, for example, detects an extremely minute analyte in a living body by applying a phenomenon (surface plasmon resonance (SPR) phenomenon) that obtains a high light output by resonance between electrons and light in a fine region at a nanometer level or the like.

In addition, a surface plasmon-field enhanced fluorescence spectroscopic apparatus (hereinafter, also referred to as "SPFS apparatus") capable of detecting an analyte with higher accuracy than the SPR apparatus on the basis of principle of surface plasmon-field enhanced fluorescence spectroscopy (SPFS) applying a surface plasmon resonance (SPR) phenomenon is also one of such sample detecting apparatuses.

This surface plasmon-field enhanced fluorescence spectroscopy (SPFS) obtains an effect of enhancing an electric field of surface plasmon light by generating surface plasmon light (compressional wave) on a surface of a metal film under a condition that excitation light such as laser light emitted from a light source undergoes attenuated total reflectance (ATR) on the surface of the metal film to increase the number of photons possessed by the excitation light emitted from the light source by dozens of times to several hundreds of times.

In such an SPFS apparatus, a sensor chip including a dielectric member, a metal film adjacent to an upper surface of the dielectric member, and a liquid holding member disposed on an upper surface of the metal film is used. In such a sensor chip, a reaction portion having a ligand for capturing an analyte is disposed on a metal film.

By supplying a sample liquid containing an analyte to the liquid holding member, the analyte is captured by the ligand (primary reaction). In this state, a liquid (labeling liquid) containing a secondary antibody labeled with a fluorescent substance is introduced into the liquid holding member. In the solution holding member, the analyte captured by the ligand is labeled with a fluorescent substance by an antigen-antibody reaction (secondary reaction).

In this state, when the metal film is irradiated with excitation light at an angle at which surface plasmon resonance occurs through the dielectric member, surface plasmon light generated on a surface of the metal film excites the fluorescent substance and the fluorescent substance generates fluorescence. By detecting this fluorescence, it is possible to measure presence or absence of the analyte and the amount thereof.

By the way, reactivity of an immune reaction such as the primary reaction or the secondary reaction generally changes depending on the temperature of a reaction field. Usually, a sample test using an SPFS apparatus is performed by setting the SPFS apparatus at room temperature. However, it is required to control the reaction field to a predetermined temperature from viewpoints of promoting an immune reaction and stabilizing reaction efficiency.

Therefore, Patent Literature 1 (JP 2012-215465 A) proposes adjustment of the temperature of a sensor chip by performing feedback control of a temperature control unit that is in contact with the sensor chip and adjusts the temperature of the sensor chip using a first temperature sensor that measures the temperature around the sensor chip and a second temperature sensor that measures the temperature of a contact portion of the sensor chip with the temperature control unit.

Specifically, the temperature of a surface of a heat transfer body of the temperature control unit in contact with the sensor chip is determined on the basis of a temperature gradient between an ambient temperature measured by the first temperature sensor and the reaction portion of the sensor chip. Then, by controlling the temperature of the surface of the heat transfer body using the determined temperature as a target value and using the temperature of the surface of the heat transfer body of the temperature control unit in contact with the sensor chip measured by the second temperature sensor as a control value, the temperature of the sensor chip is adjusted.

Citation List

Patent Literature

Patent Literature 1: JP 2012-215465 A

Summary of Invention

Technical Problem

However, in the method disclosed in Patent Literature 1, for example, in a case where the ambient temperature is low, it is intended to control the temperature of the reaction portion of the sensor chip to be constant by setting a target value to a high value. Therefore, a temperature gradient and temperature unevenness occur between a portion of the sensor chip near the temperature control unit and a portion of the sensor chip far therefrom (or a portion where heat is likely to be released).
In addition, in sample detection by a sensor chip using a fully automatic sample detecting apparatus, a liquid such as a sample liquid or a cleaning liquid is sequentially introduced into the sensor chip in the sample detecting apparatus. Therefore, the temperature of the reaction portion of the sensor chip is affected by the temperature of a liquid to be introduced.
For this reason, it is desirable to change control conditions of the temperature control unit at any time according to the temperature of a liquid to be introduced, and to control the temperature of a reaction field to be constant. However, in the case of the contact type temperature control unit used in the apparatus of Patent Literature 1, the heat capacity is large, and it is difficult to sensitively follow a change in temperature.
An object of the present invention is to provide a sample detecting system capable of suppressing a temperature gradient and temperature unevenness in a sensor chip and controlling the temperature of a reaction portion with higher accuracy regardless of the temperature of an environment in which a sample detecting apparatus is disposed.

Solution to Problem

The present invention has been made in order to solve the above-described problems in related art. In order to achieve at least one of the above-described objects, a sample detecting system reflecting one aspect of the present invention is
a sample detecting system that detects an analyte using a sensor chip internally having a reaction field that captures the analyte,
the sample detecting system including:

a contact type temperature control unit disposed in contact with the sensor chip; and
a non-contact type temperature control unit disposed in non-contact with the sensor chip, in which
the contact type temperature control unit includes a first temperature controller and a first temperature sensor that measures a temperature between the first temperature controller and the sensor chip, and performs feedback control of the first temperature controller on the basis of an output value of the first temperature sensor and a predetermined first temperature controller target temperature, and
the non-contact type temperature control unit includes a second temperature controller and a second temperature sensor that measures a temperature between the second temperature controller and the sensor chip, and performs feedback control of the second temperature controller on the basis of an output value of the second temperature sensor and a predetermined second temperature controller target temperature.

Advantageous Effects of Invention

According to the present invention, by use of a non-contact temperature controller such as warm air or cold air, a temperature gradient and temperature unevenness in the sensor chip can be suppressed, a sensitive response is possible also to a change in temperature of the sensor chip due to liquid introduction, and the temperature of the reaction portion can be controlled with high accuracy.
In addition, the temperature of an environment in which the sample detecting apparatus is disposed is measured, and a temperature target value of the non-contact temperature controller such as warm air or cold air is controlled on the basis of the environmental temperature. Therefore, the temperature of the reaction portion can be controlled with high accuracy regardless of the temperature of the environment in which the sample detecting apparatus is disposed.

Brief Description of Drawings

Fig. 1 is a schematic diagram for explaining a configuration of a surface plasmon-field enhanced fluorescence spectroscopic apparatus (SPFS apparatus) according to an embodiment of the present invention.
Fig. 2 is a flowchart illustrating an example of an operation procedure of the SPFS apparatus in the present embodiment.
Fig. 3 is a graph for explaining a relationship between elapsed time from start of sample detection and a second temperature controller target temperature T2.
Fig. 4 is a graph illustrating a relationship between elapsed time and a reaction field temperature when temperature control of a sensor chip is performed using the SPFS apparatus in the present embodiment.
Fig. 5 is a graph illustrating a relationship between elapsed time and a reaction field temperature when temperature control of a sensor chip is performed as Comparative Example 1.
Fig. 6 is a graph illustrating a relationship between elapsed time and a reaction field temperature when temperature control of a sensor chip is performed as Comparative Example 2.
Fig. 7 is a graph illustrating a relationship between elapsed time and a reaction field temperature when temperature control of a sensor chip is performed as Comparative Example 3.
Fig. 8 is a schematic view illustrating a modification of a contact type temperature control unit.
Fig. 9 is a schematic view illustrating a modification of a non-contact type temperature control unit.
Fig. 10 is a schematic view illustrating a modification of a sensor chip.

Description of Embodiments

Hereinafter, an embodiment (Example) of the present invention will be described in more detail on the basis of the drawings.

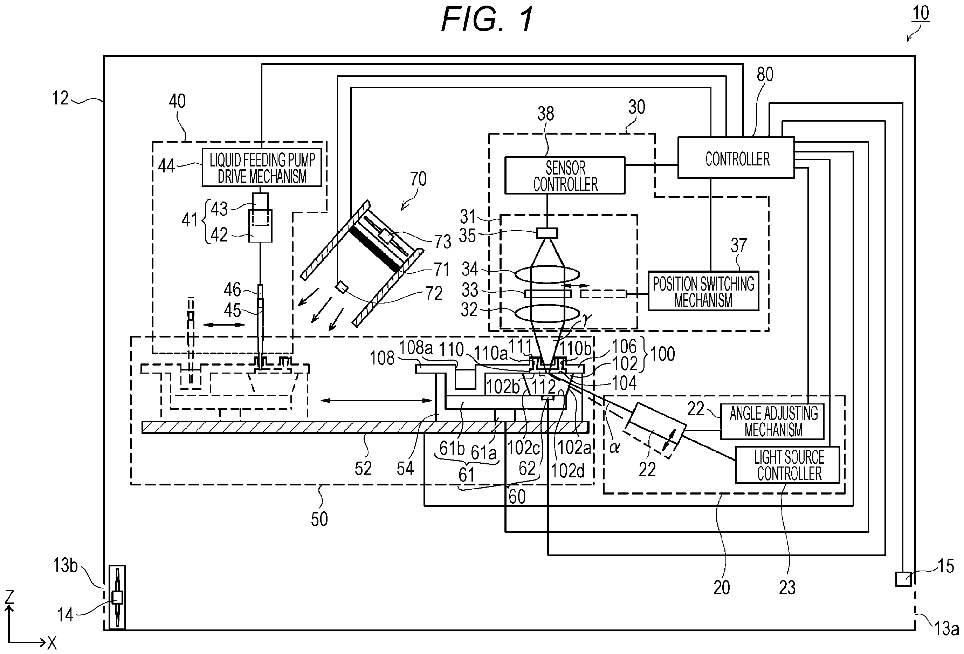

Fig. 1 is a schematic diagram for explaining a configuration of a surface plasmon-field enhanced fluorescence spectroscopic apparatus (SPFS apparatus) according to an embodiment of the present invention.
As illustrated in Fig. 1, an SPFS apparatus 10 includes an excitation light irradiation unit 20, a fluorescence detecting unit 30, a liquid feeding unit 40, a transport unit 50, a contact type temperature control unit 60, a non-contact type temperature control unit 70, and a controller 80 in a housing 12. Note that the SPFS apparatus 10 is used with a sensor chip 100 mounted on a chip holder 54 of the transport unit 50.
The sensor chip 100 includes a dielectric member 102 having an incident surface 102a, a film forming surface 102b, and an emitting surface 102c, a metal film 104 formed on the film forming surface 102b, a channel forming member 106 that is a liquid holding member fixed onto the film forming surface 102b or the metal film 104, and a liquid storage member 108 in which a sample liquid, a labeling liquid, a cleaning liquid, and the like are stored. Usually, the sensor chip 100 is exchanged for each sample test.
The sensor chip 100 is a structure preferably having each side of several mm to several cm, but may be a smaller or larger structure not included in a category of "chip".
The dielectric member 102 can be a prism made of a dielectric that is transparent to excitation light $\alpha$. The incident surface 102a of the dielectric member 102 is a surface on which excitation light $\alpha$ emitted from the excitation light irradiation unit 20 is incident on the inside of the dielectric member 102. The metal film 104 is formed on the film forming surface 102b. Excitation light $\alpha$ incident on the inside of the dielectric member 102 is reflected on an interface between the metal film 104 and the film forming surface 102b of the dielectric member 102 (hereinafter referred to as "back surface of the metal film 104" for convenience), and emitted to the outside of the dielectric member 102 through the emitting surface 102c.
The shape of the dielectric member 102 is not particularly limited. The dielectric member 102 illustrated in Fig. 1 is a prism formed of a hexahedron (truncated square pyramid shape) having a substantially trapezoidal vertical cross section. However, for example, the dielectric member 102 may be a prism having a triangular vertical cross section (so-called triangular prism), a semicircular vertical cross section, or a semielliptical vertical cross section.
The incident surface 102a is formed such that excitation light $\alpha$ does not return to the excitation light irradiation unit 20. When a light source of excitation light $\alpha$ is, for example, a laser diode (hereinafter, also referred to as "LD"), return of excitation light $\alpha$ to the LD disturbs an excitation state of the LD, and causes a wavelength of excitation light $\alpha$ or an output thereof to fluctuate. Therefore, an angle of the incident surface 102a is set such that excitation light $\alpha$ is not vertically incident on the incident surface 102a in a scanning range around an ideal enhancement angle.
Note that a design of the sensor chip 100 generally determines a resonance angle (and an enhancement angle extremely close thereto). Examples of a design element include the refractive index of the dielectric member 102, the refractive index of the metal film 104, the film thickness of the metal film 104, the extinction coefficient of the metal film 104, and the wavelength of excitation light α. An analyte immobilized on the metal film 104 shifts the resonance angle and the enhancement angle, but the amount thereof is less than a few degrees.

The dielectric member 102 has not a little birefringence characteristic. Examples of a material of the dielectric member 102 include various inorganic substances such as glass and ceramics, a natural polymer, and a synthetic polymer. A material having excellent chemical stability, manufacturing stability, optical transparency, and low birefringence is preferable.

The material is not particularly limited as described above as long as being made of a material at least optically transparent to excitation light α and having low birefringence. However, the dielectric member 102 is preferably made of, for example, a resin material in order to provide the inexpensive sensor chip 100 having excellent handleability. Note that a method for manufacturing the dielectric member 102 is not particularly limited, but injection molding using a die is preferable from a viewpoint of manufacturing cost.

When the dielectric member 102 is made of a resin material, examples of the resin material include a polyolefin such as polyethylene (PE) or polypropylene (PP), a polycyclic olefin such as a cyclic olefin copolymer (COC) or a cyclic olefin polymer (COP), polystyrene, polycarbonate (PC), an acrylic resin, and triacetyl cellulose (TAC).

The metal film 104 is formed on the film forming surface 102b of the dielectric member 102. As a result, an interaction (surface plasmon resonance) occurs between photons of excitation light α incident on the film forming surface 102b under total reflection conditions and free electrons in the metal film 104, and localized field light can be generated on a surface of the metal film 104.

A material of the metal film 104 is not particularly limited as long as being a metal capable of causing surface plasmon resonance, and is for example, made of at least one metal selected from the group consisting of gold, silver, aluminum, copper, and platinum, preferably made of gold, and furthermore, may be made of an alloy of these metals. These metals are suitable for the metal film 104 because of being stable to oxidation and increasing electric field enhancement due to surface plasmon light.

In addition, a method for forming the metal film 104 is not particularly limited, but examples thereof include a sputtering method, a vapor deposition method (a resistance heating vapor deposition method, an electron beam vapor deposition method, or the like), electrolytic plating, and an electroless plating method. The sputtering method or the vapor deposition method is desirably used because of easiness in adjusting metal film forming conditions.

The thickness of the metal film 104 is not particularly limited, but is preferably within a range of 5 to 500 nm. The thickness of the metal film 104 is more preferably within a range of 20 to 70 nm in a case of gold, silver, copper, or platinum, within a range of 10 to 50 nm in a case of aluminum, and within a range of 10 to 70 nm in a case of an alloy thereof from a viewpoint of an electric field enhancing effect.

The thickness of the metal film 104 within the above range is preferable because surface plasmon light is easily generated. In addition, the dimensions (length x width) and shape of the metal film 104 are not particularly limited as long as the metal film 104 has such a thickness.

Although not illustrated in Fig. 1, a ligand for capturing an analyte is immobilized on a surface of the metal film 104 not facing the dielectric member 102 (hereinafter referred to as "surface of the metal film 104" for convenience). By immobilizing the ligand, an analyte can be selectively detected.

In the present embodiment, a ligand is uniformly immobilized on the metal film 104 in a predetermined region (reaction field). The type of ligand is not particularly limited as long as being able to capture an analyte. In the present embodiment, the ligand is an antibody specific to an analyte or a fragment thereof.

The channel forming member 106 is disposed on the film forming surface 102b of the dielectric member 102 or the metal film 104. In the channel forming member 106, a channel groove 110 is formed on a surface facing the film forming surface 102b or the metal film 104. The channel forming member 106 is disposed so as to cover a reaction field on the metal film 104. The channel forming member 106 and the dielectric member 102 form a channel 112 for feeding a sample liquid, a labeling liquid, a cleaning liquid, or the like.

Note that the channel forming member 106 can be bonded to the dielectric member 102 or the metal film 104 by, for example, adhesion using an adhesive or a transparent adhesive sheet, laser welding, ultrasonic welding, or pressure bonding using a clamp member. By bonding the channel forming member 106 to the dielectric member 102 or the metal film 104 using an adhesive sheet having a through hole therein without forming the channel groove 110, the through hole of the adhesive sheet can also be used as a channel.

The channel forming member 106 has a first through hole 110a formed at one end of the channel groove 110 and a second through hole 110b formed at the other end of the channel groove 110. In the present embodiment, each of the first through hole 110a and the second through hole 110b has a substantially cylindrical shape. The first through hole 110a and the second through hole 110b function as inlets for injecting a sample liquid, a labeling liquid, a cleaning liquid, or the like into the channel 112, and outlets for taking out a sample liquid, a labeling liquid, a cleaning liquid, or the like.

A material of the channel forming member 106 is not particularly limited as long as being at least optically transparent to fluorescence γ described later. However, the channel forming member 106 is preferably made of, for example, a resin material in order to provide the inexpensive sensor chip 100 having excellent handleability. Note that the method for manufacturing the channel forming member 106 is not particularly limited, but injection molding using a die is preferable from a viewpoint of manufacturing cost.

When the channel forming member 106 is made of a resin material, examples thereof include a polyester such as polyethylene terephthalate (PET) or polyethylene naphthalate, a polyolefin such as polyethylene (PE) or polypropylene (PP), a polycyclic olefin such as a cyclic olefin copolymer (COC) or a cyclic olefin polymer (COP), a vinyl-based resin such as polyvinyl chloride or polyvinylidene chloride, polystyrene, polyetheretherketone (PEEK), polysulfone (PSF), polyethersulfone (PES), polycarbonate (PC), polyamide, polyimide, an acrylic resin, and triacetylcellulose (TAC).

The liquid storage member 108 includes a well 108a for storing a sample liquid, a labeling liquid, a cleaning liquid, or the like. In the present embodiment, the liquid storage member 108 is integrally formed with the channel forming member 106.

The well 108a of the liquid storage member 108 stores each of a sample liquid, a labeling liquid, a cleaning liquid, and the like used in a primary reaction and a secondary reaction described later. The shape of the well 108a is not particularly limited, and can be appropriately set according to the amount of a sample liquid, a labeling liquid, a cleaning liquid, or the like to be stored. Although only one well 108a is illustrated in the SPFS apparatus 10 illustrated in Fig. 1, a plurality of wells 108a can be disposed according to the number of liquids used in sample detection.

Note that the liquid storage member 108 and the channel forming member 106 are integrally formed in the present embodiment, but the liquid storage member 108 can be formed as a separate chip from the channel forming member 106.

A material of the liquid storage member 108 is not particularly limited. However, the liquid storage member 108 is preferably made of, for example, a resin material in order to provide the inexpensive sensor chip 100 having excellent handleability. Note that a method for manufacturing the liquid storage member 108 is not particularly limited, but injection molding using a die is preferable from a viewpoint of manufacturing cost.

When the liquid storage member 108 is made of a resin material, examples thereof include a polyester such as polyethylene terephthalate (PET) or polyethylene naphthalate, a polyolefin such as polyethylene (PE) or polypropylene (PP), a polycyclic olefin such as a cyclic olefin copolymer (COC) or a cyclic olefin polymer (COP), a vinyl-based resin such as polyvinyl chloride or polyvinylidene chloride, polystyrene, polyetheretherketone (PEEK), polysulfone (PSF), polyethersulfone (PES), polycarbonate (PC), polyamide, polyimide, an acrylic resin, and triacetylcellulose (TAC).

The sensor chip 100 having such a configuration as described above is mounted on the chip holder 54 of the transport unit 50 of the SPFS apparatus 10 as illustrated in Fig. 1, and the SPFS apparatus 10 performs sample detection.

Next, components of the SPFS apparatus 10 will be described. As described above, the SPFS apparatus 10 in the present embodiment includes the excitation light irradiation unit 20, the fluorescence detecting unit 30, the liquid feeding unit 40, the transport unit 50, the contact type temperature control unit 60, the non-contact type temperature control unit 70, and the controller 80 in the housing 12.

The excitation light irradiation unit 20 irradiates the sensor chip 100 held by the chip holder 54 with excitation light $\alpha$. As described later, when fluorescence $\gamma$ is measured, the excitation light irradiation unit 20 emits only P-wave with respect to the metal film 104 toward the incident surface 102a such that an incident angle thereof with respect to the metal film 104 becomes an angle to cause surface plasmon resonance.

Here, the "excitation light" is light to excite a fluorescent substance directly or indirectly. For example, excitation light $\alpha$ is light to generate localized field light to excite a fluorescent substance on a surface of the metal film 104 when the metal film 104 is irradiated with excitation light $\alpha$ through the dielectric member 102 at an angle at which surface plasmon resonance occurs.

The excitation light irradiation unit 20 includes a configuration for emitting excitation light $\alpha$ toward the dielectric member 102 and a configuration for performing scanning for an incident angle of excitation light $\alpha$ with respect to the back surface of the metal film 104. In the present embodiment, the excitation light irradiation unit 20 includes a light source unit 21, an angle adjusting mechanism 22, and a light source controller 23.

The light source unit 21 emits the collimated excitation light $\alpha$ having a constant wavelength and light quantity such that an irradiation spot on a back surface of the metal film 104 has a substantially circular shape. The light source unit 21 includes, for example, a light source of excitation light $\alpha$, a beam shaping optical system, an automatic power-control (APC) mechanism, and a temperature adjusting mechanism (none of which are illustrated).

The type of light source is not particularly limited, and examples thereof include a laser diode (LD), a light emitting diode, a mercury lamp, and other laser light sources. When light emitted from a light source is not a beam, the light emitted from the light source is converted into a beam by a lens, a mirror, a slit, or the like. When light emitted from a light source is not monochromatic light, the light emitted from the light source is converted into monochromatic light by a diffraction grating or the like. When light emitted from a light source is not linearly polarized light, the light emitted from the light source is converted into linearly polarized light by a polarizer or the like.

For example, the beam shaping optical system includes a collimator, a bandpass filter, a linearly polarizing filter, a half-wave plate, a slit, and a zooming means. The beam shaping optical system may include all of these means or may include only a part thereof.

The collimator collimates excitation light $\alpha$ emitted from a light source. The bandpass filter converts excitation light $\alpha$ emitted from a light source into narrow band light having only a center wavelength. This is because excitation light $\alpha$ from a light source has a small wavelength distribution width.

The linearly polarizing filter converts excitation light $\alpha$ emitted from a light source into completely linearly polarized light. The half-wave plate adjusts a polarization direction of excitation light $\alpha$ such that a P-wave component is incident on the metal film 104. The slit and zooming means adjust a beam diameter of excitation light $\alpha$, a contour shape thereof, and the like such that an irradiation spot on the back surface of the metal film 104 has a circular shape having a predetermined size.

The APC mechanism controls a light source such that an output of the light source is constant. More specifically, the APC mechanism detects a light quantity of light branching from excitation light $\alpha$ with a photodiode (not illustrated) or the like. Then, the APC mechanism controls an output of a light source constantly by controlling input energy in a regression circuit.

Examples of the temperature adjusting mechanism include a heater and a Peltier element. A wavelength and energy of light emitted from a light source may vary depending on a temperature. Therefore, by keeping the temperature of a light source constantly by the temperature adjusting mechanism, the wavelength and energy of light emitted from a light source are controlled constantly.

The angle adjusting mechanism 22 adjusts an incident angle of excitation light $\alpha$ on the metal film 104. The angle adjusting mechanism 22 rotates an optical axis of excitation light $\alpha$ and the chip holder 54 relatively in order to emit excitation light $\alpha$ toward a predetermined position of the metal film 104 through the dielectric member 102 at a predetermined incident angle.

For example, the angle adjusting mechanism 22 rotates the light source unit 21 around an axis (axis perpendicular to paper face of Fig. 1) perpendicular to the optical axis of excitation light $\alpha$. At this time, a position of the rotational axis is set such that a position of an irradiation spot on the metal film 104 is hardly changed even when scanning for an incident angle is performed. By setting the position of the rotation center to the vicinity of an intersection of the two optical axes of excitation light $\alpha$ at both ends of a scanning range for an incident angle (between the irradiation position on the film forming surface 102b and the incident surface 102a), deviation of the irradiation position can be minimized.

Among incident angles of excitation light $\alpha$ with respect to the metal film 104, an angle at which a light quantity of plasmon scattered light $\delta$ is maximum is an enhancement angle. By setting an incident angle of excitation light $\alpha$ to the enhancement angle or an angle in the vicinity thereof, fluorescence $\gamma$ having a high intensity can be measured.

Note that basic incident conditions of excitation light $\alpha$ are determined by a material of the dielectric member 102 of the sensor chip 100 and a shape thereof, the film thickness of the metal film 104, the refractive index of a sample liquid in the channel 112, and the like. However, an optimum incident condition varies slightly depending on the type of analyte in the channel 112, the amount thereof, a shape error of the dielectric member 102, and the like. Therefore, an optimum enhancement angle is preferably determined for each sample test.

The light source controller 23 controls various devices included in the light source unit 21 to control emission of excitation light $\alpha$ from the light source unit 21. For example, the light source controller 23 is constituted by a known computer or microcomputer including a computing device, a control device, a storage device, an input device, and an output device.

The fluorescence detecting unit 30 detects fluorescence $\gamma$ generated from a fluorescent substance excited by irradiation with excitation light $\alpha$ to the metal film 104. In addition, the fluorescence detecting unit 30 also detects plasmon scattered light generated by irradiation with excitation light $\alpha$ to the metal film 104, if necessary. The fluorescence detecting unit 30 includes, for example, a light receiving unit 31, a position switching mechanism 37, and a sensor controller 38.

The light receiving unit 31 is disposed in a normal direction (z-axis direction in Fig. 1) of the metal film 104 of the sensor chip 100. The light receiving unit 31 includes a first lens 32, an optical filter 33, a second lens 34, and a light receiving sensor 35.

For example, the first lens 32 is a condenser lens, and condenses light generated from an upper surface of the metal film 104. For example, the second lens 34 is an imaging lens, and images the light condensed by the first lens 32 on a light receiving surface of the light receiving sensor 35. Optical paths between the two lenses 32 and 34 are substantially parallel to each other. The optical filter 33 is disposed between the two lenses 32 and 34.

The optical filter 33 leads only a fluorescence component to the light receiving sensor 35, and removes an excitation light component (plasmon scattered light) in order to detect fluorescence $\gamma$ at a high S/N ratio. Examples of the optical filter 33 include an excitation light reflection filter, a short wavelength cut filter, and a bandpass filter. For example, the optical filter 33 is a filter including a multilayer film for reflecting a predetermined light component, but may be a colored glass filter for absorbing a predetermined light component.

The light receiving sensor 35 detects fluorescence $\gamma$. The light receiving sensor 35 is not particularly limited as long as having high sensitivity so as to be able to detect the weak fluorescence $\gamma$ from a fluorescent substance labeled with a minute amount of analyte, but examples thereof include a photomultiplier tube (PMT), an avalanche photodiode (APD), and a low noise pholo diode (PD).

The position switching mechanism 37 switches a position of the optical filter 33 between a position on an optical path and a position outside the optical path in the light receiving unit 31. Specifically, the optical filter 33 is disposed on the optical path of the light receiving unit 31 when the light receiving sensor 35 detects fluorescence $\gamma$, and the optical filter 33 is disposed outside the optical path of the light receiving unit 31 when the light receiving sensor 35 detects plasmon scattered light. The position switching mechanism 37 is constituted by, for example, a rotational drive unit and a known mechanism (a turntable, a rack and pinion, or the like) for moving the optical filter 33 in the horizontal direction using rotational movement.

The sensor controller 38 controls detection of an output value of the light receiving sensor 35, management of a sensitivity of the light receiving sensor 35 with the detected output value, change of the sensitivity of the light receiving sensor 35 for obtaining a proper output value, and the like. The sensor controller 38 is constituted by, for example, a known computer or microcomputer including a computing device, a control device, a storage device, an input device, and an output device.

The liquid feeding unit 40 supplies a sample liquid, a labeling liquid, a cleaning liquid, or the like into the channel 112 of the sensor chip 100 mounted on the chip holder 54. The liquid feeding unit 40 includes a syringe pump 41, a pipette nozzle 46, a pipette tip 45, and a liquid feeding pump drive mechanism 44.

The liquid feeding unit 40 is used with the pipette tip 45 mounted on a tip of the pipette nozzle 46. If the pipette tip 45 is replaceable, cleaning of the pipette tip 45 is unnecessary, and contamination of impurities or the like can be prevented.

The syringe pump 41 is constituted by a syringe 42 and a plunger 43 capable of being reciprocated in the syringe 42. By the reciprocating motion of the plunger 43, suction of a liquid and ejection thereof are performed quantitatively.

The liquid feeding pump drive mechanism 44 includes a syringe pump 41 driving device and a pipette nozzle 46 moving device on which the pipette tip 45 is mounted. The syringe pump 41 driving device is a device for reciprocating the plunger 43, and for example, includes a stepping motor. A driving device including a stepping motor can manage a liquid feeding amount of the syringe pump 41 or a liquid feeding rate thereof, and is therefore preferable from a viewpoint of managing the amount of a residual liquid in the sensor chip 100. The pipette nozzle 46 moving device freely moves the pipette nozzle 46, for example, in two directions of an axial direction of the pipette nozzle 46 (for example, vertical direction) and a direction crossing the axial direction (for example, horizontal direction). The pipette nozzle 46 moving device is constituted by, for example, a robot arm, a 2-axis stage, or a vertically movable turntable.

The liquid feeding unit 40 preferably further includes a mechanism for detecting the position of a tip of the pipette tip 45 from a viewpoint of adjusting a relative height between the pipette tip 45 and the sensor chip 100 to a constant level and managing the amount of a residual liquid in the sensor chip 100 at a constant level.

The liquid feeding unit 40 sucks various liquids from the liquid storage member 108, and supplies the liquids into the channel 112 of the sensor chip 100. At this time, by moving the plunger 43, a liquid is reciprocated in the channel 112 of the sensor chip 100, and the liquid in the channel 112 is stirred. This can make the concentration of the liquid uniform, or can accelerate a reaction in the channel 112 (for example, an antigen-antibody reaction), for example.

Since such an operation is performed, preferably, the inlet (first through hole 110a) of the sensor chip 100 is protected by a multilayer film 111 or the like, and the sensor chip 100 and the pipette tip 45 are configured so as to be able to seal the first through hole 110a when the pipette tip 45 passes though the multilayer film.

The liquid in the channel 112 is sucked by the syringe pump 41 again, and is discharged to the liquid storage member 108 or the like. By repeating these operations, a reaction of various liquids, cleaning, and the like are performed, and an analyte labeled with a fluorescent substance can be immobilized on a reaction field in the channel 112.

The transport unit 50 transports the sensor chip 100 mounted on the chip holder 54 by a user to a liquid feeding position or a measurement position and fixes the sensor chip 100 thereto. Here, the "liquid feeding position" is a position at which the liquid feeding unit 40 supplies a liquid into the channel 112 of the sensor chip 100 or removes the liquid in the channel 112. The "measurement position" is a position at which the excitation light irradiation unit 20 irradiates the sensor chip 100 with excitation light $\alpha$ and the fluorescence detecting unit 30 detects fluorescence $\gamma$ generated in accordance therewith.

Note that the transport unit 50 is also used for changing a distance between the sensor chip 100 and the light source unit 21 of the excitation light irradiation unit 20 in a position detection and position adjustment step described later.

The transport unit 50 includes a transport stage 52 and the chip holder 54. The chip holder 54 is fixed to the transport stage 52, and holds the sensor chip 100 detachably. The shape of the chip holder 54 is not particularly limited as long as being able to hold the sensor chip 100 and having a shape not interfering with the optical paths of excitation light $\alpha$ and fluorescence $\gamma$. For example, the chip holder 54 has an opening through which excitation light $\alpha$ and fluorescence $\gamma$ pass.

The transport stage 52 can move the chip holder 54 in one direction (x-axis direction in Fig. 1) and the opposite direction. The transport stage 52 is driven, for example, by a stepping motor.

The contact type temperature control unit 60 includes a first temperature controller 61 disposed in contact with the sensor chip 100, and a first temperature sensor 62 disposed between the first temperature controller 61 and a reaction field of the sensor chip 100.

The first temperature controller 61 is controlled by the controller 80 described later so as to have a predetermined temperature. In the present embodiment, the first temperature controller 61 includes a temperature control element 61a and a heat transfer member 61b.

The temperature control element 61a may be a heating element or a cooling element. The temperature control element 61a is not particularly limited, but examples thereof include an electric resistance element, a cartridge heater, a rubber heater, an infrared heater such as a ceramic heater, and a Peltier element.

The heat transfer member 61b has a shape not interfering with the optical paths of excitation light $\alpha$ and fluorescence $\gamma$, and transfers heat from the temperature control element 61a to the sensor chip 100. A material of the heat transfer member 61b is not particularly limited, but examples thereof include a metal having a high thermal conductivity such as copper or aluminum.

In the present embodiment, the heat transfer member 61b has such a shape to be in contact with a lower surface 102d of the dielectric member 102 and also to be in contact with the well 108a of the liquid storage member 108. This makes it possible to perform temperature control of a liquid stored in the well 108a of the liquid storage member 108 simultaneously with temperature control of the reaction field of the sensor chip 100. Therefore, temperature control of a liquid introduced into the channel 112 of the sensor chip 100 can be performed in advance, and a change in temperature of the reaction field caused by the introduction of the liquid into the channel 112 can be suppressed.

The first temperature sensor 62 is not particularly limited as long as being able to transmit a signal (output value) corresponding to a measured temperature to the controller 80 described later, but examples thereof include a thermistor and a thermocouple.

The first temperature sensor 62 may be located at any position where a temperature can be measured between the first temperature controller 61 and the sensor chip 100, and is preferably disposed closer to the sensor chip 100 having a reaction field. For example, the first temperature sensor 62 may be disposed at a position in contact with any surface of the dielectric member 102 on the heat transfer member 61b or in the heat transfer member 61b close to the dielectric member 12.

The non-contact type temperature control unit 70 includes a second temperature controller 71 disposed apart from the sensor chip 100, a second temperature sensor 72 disposed between the second temperature controller 71 and the sensor chip 100, and an air blower 73 that sends air heated or cooled by the second temperature controller 71 to the sensor chip 100. In the present embodiment, the non-contact type temperature control unit 70 is disposed such that temperature control of the sensor chip 100 is possible in a state where the sensor chip 100 is at a liquid feeding position.

The second temperature controller 71 is controlled by the controller 80 described later so as to have a predetermined temperature. The second temperature controller 71 may be a heating element or a cooling element. The second temperature controller 71 is not particularly limited, but examples thereof include an electric resistance element, a cartridge heater, a rubber heater, an infrared heater such as a ceramic heater, and a Peltier element.

Air heated or cooled by the second temperature controller 71 is blown on the sensor chip 100 by the air blower 73. As a result, the sensor chip 100 is heated or cooled in a non-contact manner. The air blower 73 is not particularly limited, but examples thereof include a well-known fans such as an axial-flow fan or a centrifugal fan. Note that the air blower 73 can preferably change a pressure ratio by the controller 80 described later.

The second temperature sensor 72 is not particularly limited as long as being able to transmit a signal (output value) corresponding to a measured temperature to the controller 80 described later, but examples thereof include a thermistor and a thermocouple. Note that the second temperature sensor 72 measures the temperature of air blown on the sensor chip 100.

The controller 80 controls the angle adjusting mechanism 22, the light source controller 23, the position switching mechanism 37, the sensor controller 38, the transport stage 52, the first temperature controller 61, the second temperature controller 71, and the air blower 73. For example, the controller 80 is constituted by a known computer or microcomputer including a computing device, a control device, a storage device, an input device, and an output device.

The housing 12 is not particularly limited as long as being able to house the excitation light irradiation unit 20, the fluorescence detecting unit 30, the liquid feeding unit 40, the transport unit 50, the contact type temperature control unit 60, the non-contact type temperature control unit 70, and the controller 80 therein. The housing 12 has an intake port 13a and an exhaust port 13b therein. This makes it possible to prevent heat from being accumulated in the housing 12. A fan 14 is preferably disposed in the intake port 13a and/or the exhaust port 13b from a viewpoint of exhaust heat. As a result, air in the housing 12 is discharged, and air outside the housing 12 is sucked. Heat can be thereby prevented from being accumulated in the housing 12.

The intake port 13a of the housing 12 has a third temperature sensor 15 therein. As described above, by disposing the third temperature sensor 15 in the intake port 13a, the third temperature sensor 15 can measure the temperature of an environment in which the SPFS apparatus 10 is disposed (the temperature outside the housing 12).

The third temperature sensor 15 is not particularly limited as long as being able to transmit a signal (output value) corresponding to a measured temperature to the controller 80, but examples thereof include a thermistor and a thermocouple. Note that the third temperature sensor 15 only needs to be able to measure the temperature of an environment in which the SPFS apparatus 10 is disposed. In the present embodiment, the third temperature sensor 15 is disposed in the housing 12, but can also be disposed outside the housing 12.

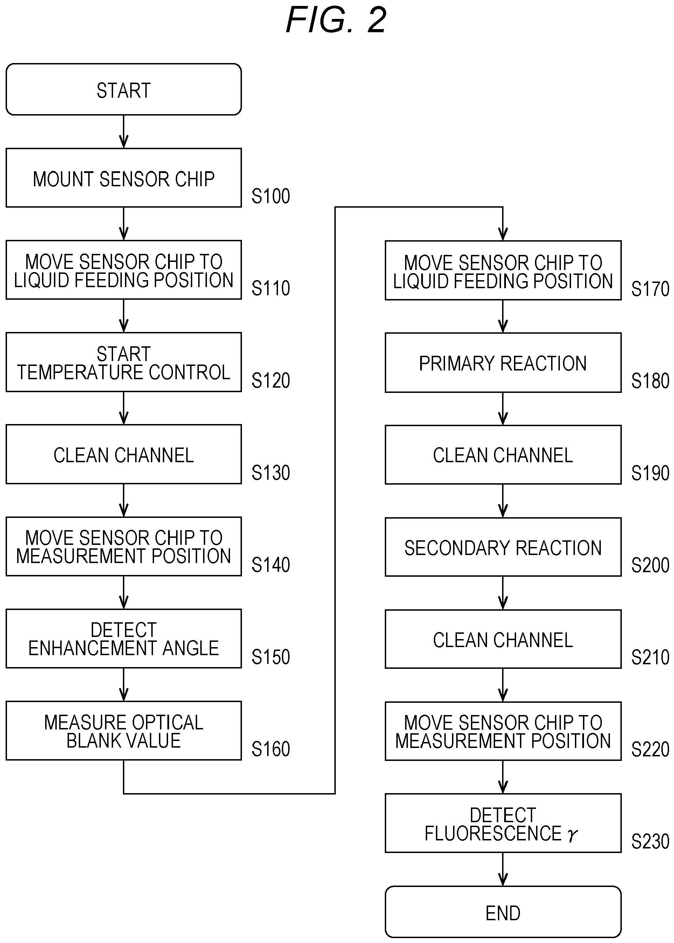

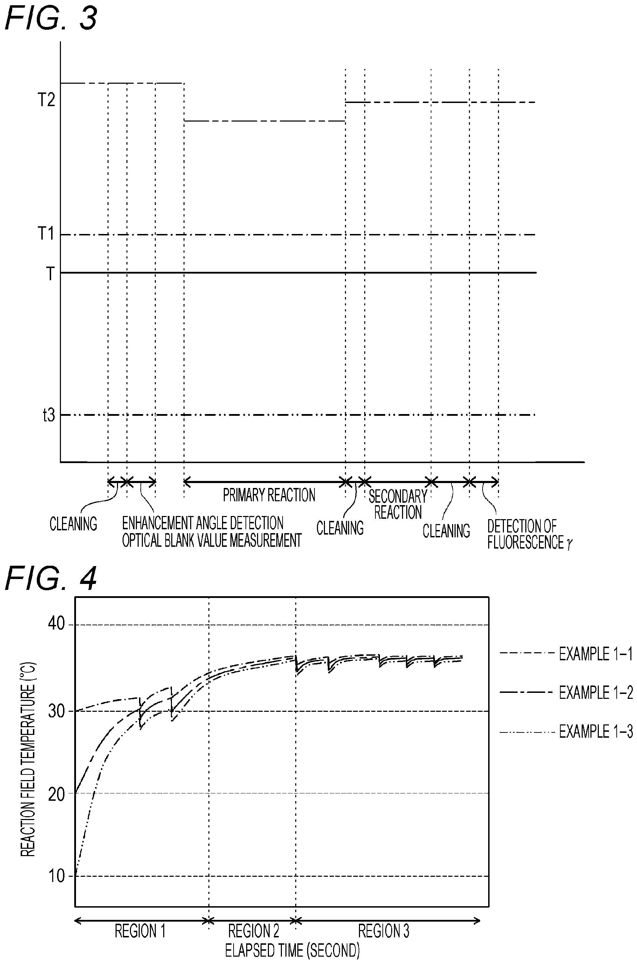

Hereinafter, a flow of sample detection using the SPFS apparatus 10 will be described. Fig. 2 is a flowchart illustrating an example of an operation procedure of the SPFS apparatus 10. Fig. 3 is a graph for explaining a relationship between elapsed time from start of sample detection and a second temperature controller target temperature $T2$.

First, a user mounts the sensor chip 100 in which a sample liquid containing an analyte, a labeling liquid, a cleaning liquid, or the like is stored in the liquid storage member 108 on the chip holder 54 of the transport unit 50 (S100).

Note that the sample liquid used here is a liquid prepared using a sample, and examples thereof include a liquid obtained by mixing a sample and a reagent and subjecting the resulting mixture to a treatment for bonding a fluorescent substance to an analyte contained in the sample. Examples of the sample include blood, serum, plasma, urine, nasal fluid, saliva, stool, and body cavity fluid (such as spinal fluid, ascites fluid, or pleural effusion).

Examples of the analyte contained in the sample include a nucleic acid (single-stranded or doublestranded DNA, RNA, polynucleotide, oligonucleotide, or peptide nucleic acid (PNA), or nucleoside, nucleotide, and modified molecules thereof), a protein (polypeptide or oligopeptide), an amino acid (including a modified amino acid), a carbohydrate (oligosaccharide, polysaccharide, or sugar chain), a lipid, modified molecules thereof, and complexes thereof. Specifically, the analyte may be a carcinoembryonic antigen such as $\alpha$-fetoprotein (AFP), a tumor marker, a signal transduction substance, a hormone, or the like without particular limitation.

The controller 80 operates the transport stage 52 to move the sensor chip 100 mounted on the chip holder 54 to a liquid feeding position (S110).

Subsequently, the controller 80 operates the contact type temperature control unit 60 and the non-contact type temperature control unit 70 to start temperature control of the sensor chip 100 (S120). Temperature control of the sensor chip 100 is performed as follows.

The controller 80 stores a reaction field target temperature $T$, a first temperature controller target temperature $T1$, and the second temperature controller target temperature $T2$ in advance.

The reaction field target temperature $T$ is appropriately changed depending on the type of ligand immobilized on a reaction field, the type of analyte, and the like. The reaction field target temperature $T$ can be set to 24°C to 26°C or 35°C to 37°C in general. In the present embodiment, the reaction field target temperature $T$ is 36°C. The SPFS apparatus 10 is disposed at room temperature. In the present embodiment, an environmental temperature $t3$ is 25°C.

In the present embodiment, the contact type temperature control unit 60 performs temperature control of the reaction field of the sensor chip 100 mainly by heat transfer through a contact portion.

The first temperature controller target temperature $T1$ is desirably set to a temperature close to the reaction field target temperature $T$. A difference $|T - T1|$ between the first temperature controller target temperature $T1$ and the reaction field target temperature $T$ can be appropriately set in consideration of a temperature gradient of heat transfer depending on the materials and shapes of the dielectric member 102 and the heat transfer member 61b. By setting the reaction field target temperature $T$ and the first temperature controller target temperature $T1$ so as to be close to each other while the difference $|T - T1|$ between the first temperature controller target temperature $T1$ and the reaction field target temperature $T$ is about a temperature gradient of heat transfer, it is possible to prevent excessive temperature control of the sensor chip 100 by the first temperature controller 61, and to stably control the reaction field to the reaction field target temperature $T$. In the present embodiment, the first temperature controller target temperature $T1$ is 36.5°C, and the difference $|T - T1|$ between the first temperature controller target temperature $T1$ and the reaction field target temperature $T$ is 0.5°C.

The non-contact type temperature control unit 70 blows warm air, cold air, or the like on the sensor chip 100 to mainly control an environmental temperature of a portion where the sensor chip 100 is disposed, and thereby performs temperature control of a reaction field more stably due to an effect of performing temperature control of the sensor chip 100 and an effect of controlling the amount of heat released from the sensor chip 100. Furthermore, the non-contact type temperature control unit 70 also performs temperature control of a liquid such that the temperature of a liquid introduced into the channel 112 is brought close to the reaction field target temperature $T$ as much as possible during introduction or reciprocation.

The second temperature controller target temperature $T2$ can be changed for each step of sample detection as described later. As an initial value of the second temperature controller target temperature $T2$, in order to quickly bring the temperature of the reaction field of the sensor chip 100 close to the reaction field target temperature $T$, a difference $|T - T2|$ between the second temperature controller target temperature $T2$ and the reaction field target temperature $T$ is preferably larger. In the present embodiment, the second temperature controller target temperature $T2$ is within a range of 37°C to 41°C as described later.

Incidentally, when a difference $|T - T3|$ between a temperature measured by the third temperature sensor 15 and the reaction field target temperature T is large, by making the difference |T - T2| between the second temperature controller target temperature T2 and the reaction field target temperature T larger, the temperature of the reaction field of the sensor chip 100 can be brought closer to the reaction field target temperature T quickly.

The initial value of the second temperature controller target temperature T2 can be set so as to satisfy the following formula (1) when a temperature measured by the third temperature sensor 15 is represented by t3.

[Numerical formula 1]

$$T2 = a \times (t3)^2 + b \times t3 + c \qquad (1)$$

wherein a, b, and c each represent a predetermined coefficient.

The difference |T - T2| between the second temperature controller target temperature T2 and the reaction field target temperature T and the difference |T - T1| between the first temperature controller target temperature T1 and the reaction field target temperature T are preferably set so as to satisfy the following formula (2).

[Numerical formula 2]

$$\left| T - T1 \right| \leq \left| T - T2 \right| \qquad (2)$$

This setting makes it possible to, for example, perform temperature control of the reaction field more stably due to an effect of controlling an environmental temperature of a portion where the sensor chip 100 is disposed and controlling the amount of heat released from the sensor chip 100. When a liquid is fed into the channel 112, for example, if the temperature of the liquid to be introduced is different from the reaction field target temperature T, or if the temperature of the liquid changes in the pipette tip 45 during reciprocation, the temperature of the reaction field may be changed by liquid feeding. However, also in such a case, by performing temperature control of the liquid by the non-contact type temperature control unit 70, the temperature of the reaction field can be controlled stably.

The controller 80 performs feedback control of the first temperature controller 61 such that a temperature measured by the first temperature sensor 62 is the first temperature controller target temperature T1 on the basis of a signal (output value) from the first temperature sensor 62.

In addition, the controller 80 performs feedback control of the second temperature controller 71 and the air blower 73 such that a temperature measured by the second temperature sensor 72 is the second temperature controller target temperature T2 on the basis of a signal (output value) from the second temperature sensor 72.

With the temperature control of the sensor chip 100 performed in this manner, the controller 80 operates the liquid feeding unit 40 to introduce a cleaning liquid in the well 108c of the liquid storage member 108 into the channel 112, cleans the channel 112, and removes a storage reagent in the channel 112 (S130). The cleaning liquid used for cleaning is discharged by the liquid feeding unit 40, and a measurement liquid in the well 108d of the liquid storage member 108 is introduced into the channel 112 in place of the cleaning liquid. Note that if there is no influence on a result of enhancement angle detection (S150) in a later step, the storage reagent cleaning liquid is used also as the measurement liquid, and an enhancement angle can be measured without discharging the cleaning liquid.

Subsequently, the controller 80 operates the transport stage 52 to transfer the sensor chip 100 mounted on the chip holder 54 to a measurement position (S140). Then, the controller 80 operates the excitation light irradiation unit 20 and the fluorescence detecting unit 30, irradiates the sensor chip 100 with excitation light α, detects plasmon scattered light having the same wavelength as excitation light α, and detects an enhancement angle (S150).

Specifically, the controller 80 operates the excitation light irradiation unit 20 and performs scanning for an incident angle of excitation light α with respect to the metal film 104, and operates the fluorescence detecting unit 30 and detects plasmon scattered light. At this time, the controller 80 operates the position switching mechanism 37 and disposes the optical filter 33 outside an optical path of the light receiving unit 31. Then, the controller 80 determines an incident angle of excitation light α when the light quantity of the plasmon scattered light is maximum as an enhancement angle.

Subsequently, the controller 80 operates the excitation light irradiation unit 20 and the fluorescence detecting unit 30, irradiates the sensor chip 100 disposed at an appropriate measurement position with excitation light α, and records an output value (optical blank value) of the light receiving sensor 35 (S160).

At this time, the controller 80 operates the angle adjusting mechanism 22 and sets an incident angle of excitation light α to the enhancement angle. In addition, the controller 80 operates the position switching mechanism 37 and disposes the optical filter 33 in an optical path of the light receiving unit 31.

Specifically, the controller 80 operates the transport stage 52 and moves the sensor chip 100 to a liquid feeding position (S170).

Then, the controller 80 operates the liquid feeding unit 40, discharges the measurement liquid in the channel 112, and introduces the sample liquid in the well 108a of the liquid storage member 108 into the channel 112 (S180). In the channel 112, an analyte is captured by a reaction field on the metal film 104 by an antigen-antibody reaction (primary reaction).

As illustrated in Fig. 3, in the primary reaction step, the liquid in the channel 112 is not exchanged, and the reaction time is long. Therefore, there is sufficient time for the temperature of the sample liquid to be controlled to the reaction field target temperature T in the channel 112. Therefore, the controller 80 changes the second temperature controller target temperature T2 such that the difference |T - T2| between the second temperature controller target temperature T2 and the reaction field target temperature T is smaller than the initial value to reduce an effect of performing temperature control of the sample liquid during liquid introduction or reciprocation.

Thereafter, the sample liquid in the channel 112 is removed, and the inside of the channel 112 is cleaned with the cleaning liquid (S190). The amount of the cleaning liquid in the well 108c and the amount of the cleaning liquid introduced into the channel are larger than that used in another step. Therefore, the cleaning liquid is not easily heated in the well 108c, and introduction of the cleaning liquid easily lowers the temperature of the reaction field. For this reason, as illustrated in Fig. 3, the controller 80 changes the second temperature controller target temperature T2 such that the difference |T - T2| between the second temperature controller target temperature T2 and the reaction field target temperature T is larger than that in the primary reaction step.

As described above, by appropriately changing the second temperature controller target temperature T2 in each step of sample detection, the temperature of the reaction field can be rapidly brought close to the reaction field target temperature T, an influence of a difference in liquid amount and liquid temperature of each of various liquids introduced into the reaction field is reduced, and deviation of the temperature of the reaction field from the reaction field target temperature T can be prevented. Note that the change of the second temperature controller target temperature T2 may be performed at the timing when each step starts or in the middle of each step.

Subsequently, the controller 80 operates the liquid feeding unit 40 and introduces the labeling liquid in the well 108b of the liquid storage member 108 into the channel 112 (S200). In the channel 112, an analyte captured on the metal film 104 is labeled with a fluorescent substance by an antigen-antibody reaction (secondary reaction). Note that a liquid containing a secondary antibody labeled with a fluorescent substance can be used as the labeling liquid. Thereafter, the labeling liquid in the channel 112 is removed, the inside of the channel 112 is cleaned with a cleaning liquid, and the cleaning liquid is removed. Thereafter, a measurement liquid is introduced into the channel 112 (S210).

Subsequently, the controller 80 operates the transport stage 52 and moves the sensor chip 100 to a measurement position (S220).

Subsequently, the controller 80 operates the excitation light irradiation unit 20 and the fluorescence detecting unit 30, irradiates the sensor chip 100 disposed at the measurement position with excitation light $\alpha$, and detects fluorescence $\gamma$ emitted from a fluorescent substance to label an analyte captured by a ligand (S230). On the basis of the intensity of the detected fluorescence $\gamma$, conversion to the amount, concentration, and the like of the analyte is possible, if necessary.

By the above procedure, presence or the amount of the analyte in the sample solution can be detected.

Note that the enhancement angle detection (S150) and the optical blank value measurement (S160) are performed before the primary reaction (S180) in the present embodiment. However, the enhancement angle detection (S150) and the optical blank value measurement (S160) may be performed after the primary reaction (S180).

When the incident angle of excitation light $\alpha$ is determined in advance, the enhancement angle detection (S150) may be omitted.

In the above description, after the primary reaction (S180) that causes an analyte to react with a ligand, a secondary reaction (S200) that labels the analyte with a fluorescent substance is performed (two-step method). However, the timing at which the analyte is labeled with the fluorescent substance is not particularly limited.

For example, before the sample liquid is introduced into the channel 112, the labeling liquid is added to the sample liquid, and the analyte can be labeled with the fluorescent substance in advance. By simultaneously injecting the sample liquid and the labeling liquid into the channel 112, the analyte labeled with the fluorescent substance is captured by the ligand. In this case, the analyte is labeled with the fluorescent substance, and the analyte is captured by the ligand.

In both cases, both the primary reaction and the secondary reaction can be completed by introducing the sample solution into the channel 112 (one-step method). As described above, when the one-step method is adopted, the enhancement angle detection (S150) is performed before the antigen-antibody reaction.

In the present embodiment, as described above, the second temperature controller target temperature T2 in the primary reaction step (S180) is different from that in each of the secondary reaction step (S200) and the cleaning steps (S130, S190, and S210). More specifically, the second temperature controller target temperature T2 is changed such that the difference |T - T2| between the second temperature controller target temperature T2 and the reaction field target temperature T is larger as a liquid is introduced into the channel 112 in a shorter time.

As described above, in a step in which the liquid is introduced into the channel 112 in a short time, that is, in a step of performing liquid exchange frequently, a temperature difference between the temperature of a liquid stored in the liquid storage member 108 and the reaction field target temperature T is large. Therefore, by making the difference |T - T2| between the second temperature controller target temperature T2 and the reaction field target temperature T larger, the temperature difference can be quickly eliminated.

In addition, in the present embodiment, as described above, the controller 80 performs feedback control of the first temperature controller 61, the second temperature controller 71, and the air blower 73. However, by disposing a temperature controller in each of the contact type temperature control unit 60 and the non-contact type temperature control unit 70, each temperature controller may perform feedback control of the first temperature controller 61, the second temperature controller 71, and the air blower 73.

(Examples)

Fig. 4 is a graph illustrating a relationship between elapsed time and a reaction field temperature when temperature control of a sensor chip is performed using the SPFS apparatus in the present embodiment.

In Examples, the SPFS apparatus 10 was operated under the conditions illustrated in the following Table 1, and elapsed time and a reaction field temperature were measured.

Note that in Examples and Comparative Examples described below, the second temperature controller target temperature T2 was set so as to be changeable in each of a region from start of sample detection to the middle of a primary reaction (region 1), a region from the middle of the primary reaction to an end of the primary reaction (region 2), and a region from start of cleaning after the primary reaction to an end of sample detection (region 3).

[Table 1]

| Reaction field target temperature T | | 36±1°C | | |
|---|---|---|---|---|
| First temperature controller target temperature T1 | | 36.5°C | | |
| | | | | |
| | Temperature t3 measured by third temperature sensor (environmental temperature) | Second temperature controller target temperature T2 | | |
| | | Region 1 | Region 2 | Region 3 |
| Example 1-1 | 30°C | 37°C | 37°C | 37°C |
| Example 1-2 | 20°C | 38.5°C | 37°C | 40°C |
| Example 1-3 | 10°C | 40°C | 37°C | 41°C |

In Example 1-1, an environmental temperature is relatively high, and a difference from the reaction field target temperature T is small. Therefore, the second temperature controller target temperature T2 is constant.

In Examples 1-2 and 1-3, a difference between an environmental temperature and the reaction field target temperature T is large. Therefore, in the region 1 that is an initial stage of sample detection and the region 3 in which time required for the secondary reaction or each step such as cleaning is short, and the frequency of liquid exchange is high, the second temperature controller target temperature T2 is set to be higher than that in the region 2.

When the temperatures of reaction fields become almost the same as each other at environmental temperatures in Examples 1-1, 1-2, and 1-3, the transition from region 1 to region 2 is performed. In the region 2, the second temperature controller target temperature T2 is set to be the same.

In the present Examples, the second temperature controller target temperature T2 in each of the regions 1 and 3 is set to satisfy the following formula (1) using the temperature t3 measured by the third temperature sensor 15.
[Numerical formula 3]

$$T2 = a \times (t3)^2 + b \times t3 + c \qquad\qquad (1)$$

In the present Examples,

under conditions satisfying a = 0, b = -0.15, and c = 41.5 in the region 1, and
a = -0.01, b = 0.2, and c = 40 in the region 3, the second temperature controller target temperature T2 is set and controlled.

As described above, by changing the second temperature controller target temperature T2 depending on the environmental temperature, the temperature of the reaction field can be quickly brought close to the reaction field target temperature T, and the temperature of the reaction field can be stabilized even when liquid exchange is frequently performed.

The contact type temperature control unit 60 does not easily follow a change in a set temperature sensitively because the heat capacity of the first temperature controller 61 itself is large. However, the non-contact type temperature control unit 70 can blow warm air sensitively following a change in a set temperature on the sensor chip 100, and is preferable for changing the set temperature in the middle of a reaction.

When the SPFS apparatus 10 and the sensor chip 100 are disposed in the same environment, it can be estimated that by measuring the environmental temperature t3, the initial temperature of the sensor chip 100 introduced into the SPFS apparatus 10 is approximately the t3. Therefore, by setting the second temperature controller target temperature T2 depending on the environmental temperature, temperature control reflecting the initial temperature of the reaction field of the sensor chip 100 can be performed without directly measuring the temperature of the sensor chip 100. The temperature of the reaction field can be controlled to the reaction field target temperature T more rapidly and stably by a simple method not requiring a means for measuring the temperature of the sensor chip 100.

(Comparative Example 1)

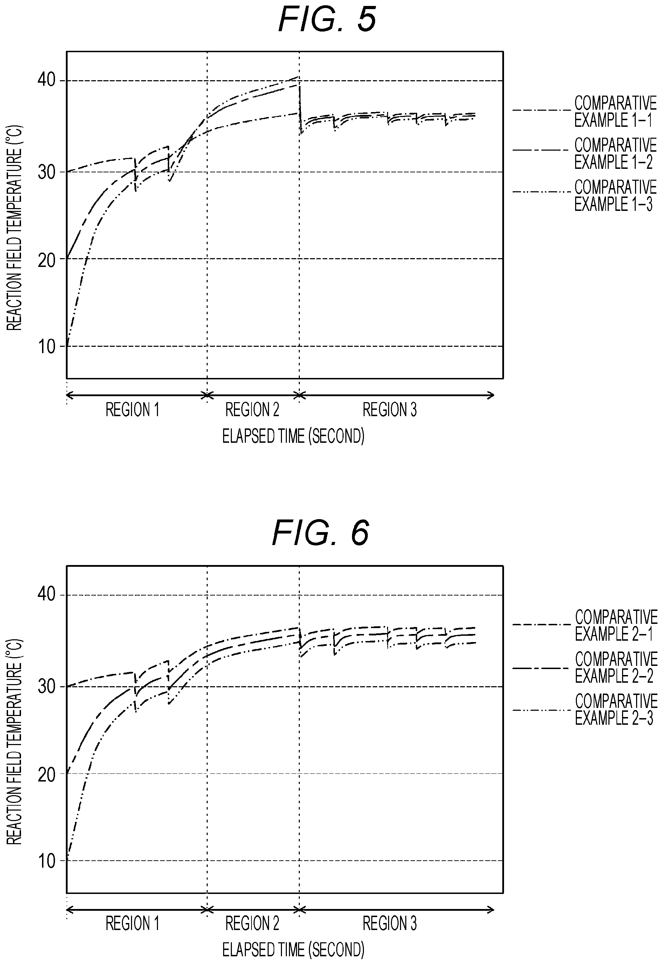

Fig. 5 is a graph illustrating a relationship between elapsed time and a reaction field temperature when temperature control of a sensor chip is performed as Comparative Example 1.

An SPFS apparatus used in Comparative Example 1 has the same apparatus configuration as the SPFS apparatus 10 in the above embodiment, and performs temperature control without changing the second temperature controller target temperature T2 in each step as illustrated in the following Table 2. A set value of the second temperature controller target temperature T2 was set to a value at which the reaction field had the reaction field target temperature T in the region 3 where the strictest temperature control was required.

[Table 2]

| Reaction field target temperature T | | 36±1°C | | |
|---|---|---|---|---|
| First temperature controller target temperature T1 | | 36.5°C | | |
| | | | | |
| | Temperature t3 measured by third temperature sensor (environmental temperature) | Second temperature controller target temperature T2 | | |
| | | Region 1 | Region 2 | Region 3 |
| Comparative Example 1-1 | 30°C | 37°C | 37°C | 37°C |
| Comparative Example 1-2 | 20°C | 40°C | 40°C | 40°C |
| Comparative Example 1-3 | 10°C | 41°C | 41°C | 41°C |

Comparative Example 1-1 had the same condition as Example 1-1, and therefore temperature control was performed without any problem.

In Comparative Examples 1-2 and 1-3, the second temperature controller target temperature T2 was relatively higher than the reaction field target temperature T. Therefore, during the primary reaction (region 2) where the reaction time was long, the temperature of the reaction field became excessively higher than the reaction field target temperature T due to excessive heating by the non-contact type temperature control unit 70.

(Comparative Example 2)

Fig. 6 is a graph illustrating a relationship between elapsed time and a reaction field temperature when temperature control of a sensor chip is performed as Comparative Example 2.

An SPFS apparatus used in Comparative Example 2 has the same apparatus configuration as the SPFS apparatus 10 in the above embodiment, and performs temperature control by keeping the second temperature controller target temperature T2 constant regardless of the environmental temperature as illustrated in the following Table 3.

[Table 3]

| Reaction field target temperature T | 36±1°C | | |
|---|---|---|---|
| First temperature controller target temperature T1 | 36.5°C | | |

| | Temperature t3 measured by third temperature sensor (environmental temperature) | Second temperature controller target temperature T2 | | |
|---|---|---|---|---|
| | | Region 1 | Region 2 | Region 3 |
| Comparative Example 2-1 | | 37°C | 37°C | 37°C |
| Comparative Example 2-2 | | 37°C | 37°C | 37°C |
| Comparative Example 2-3 | | 37°C | 37°C | 37°C |

Comparative Example 2-1 had the same condition as Example 1-1, and therefore temperature control was performed without any problem.

In each of Comparative Examples 2-2 and 2-3, the environmental temperature was lower than the reaction field target temperature T. Therefore, a temperature rising rate in each region was slower than that in Comparative Example 2-1, and the temperature of the reaction field during the secondary reaction (region 3) did not reach the reaction field target temperature T.

(Comparative Example 3)

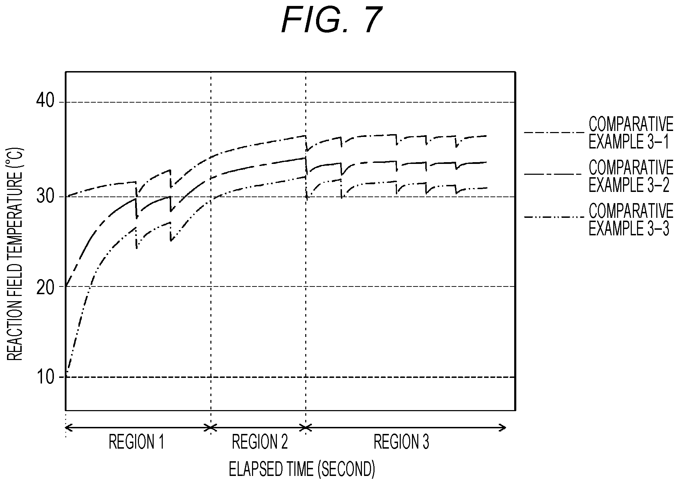

Fig. 7 is a graph illustrating a relationship between elapsed time and a reaction field temperature when temperature control of a sensor chip is performed as Comparative Example 3.

An SPFS apparatus used in Comparative Example 3 has the same apparatus configuration as the SPFS apparatus 10 in the above embodiment, and performs temperature control only with the contact type temperature control unit 60 without operating the non-contact type temperature control unit 70 as illustrated in the following Table 4.

[Table 4]

| Reaction field target temperature T | 36±1°C | | |
|---|---|---|---|
| First temperature controller target temperature T1 | 36.5°C | | |

| | Temperature t3 measured by third temperature sensor (environmental temperature) | Second temperature controller target temperature T2 | | |
|---|---|---|---|---|
| | | Region 1 | Region 2 | Region 3 |
| Comparative Example 3-1 | 30°C | - | - | - |
| Comparative Example 3-2 | 20°C | - | - | - |
| Comparative Example 3-3 | 10°C | - | - | - |

In Comparative Examples 3-1, 3-2, and 3-3, the temperature of the reaction field dropped largely at the time of liquid exchange. In addition, since the air temperature around the sensor chip 100 was low, the liquid temperature was lowered in the pipette tip 45 during reciprocation, and the amount of heat released from a surface not in contact with the contact type temperature control unit 60 was large. The temperature of the reaction field was largely different from the reaction field target temperature T in Comparative Examples 3-2 and 3-3 in which the environmental temperatures were low. In addition, a temperature gradient between a heating side and a heat release side in the sensor chip 100 was also large.

(Modification of contact type temperature control unit)

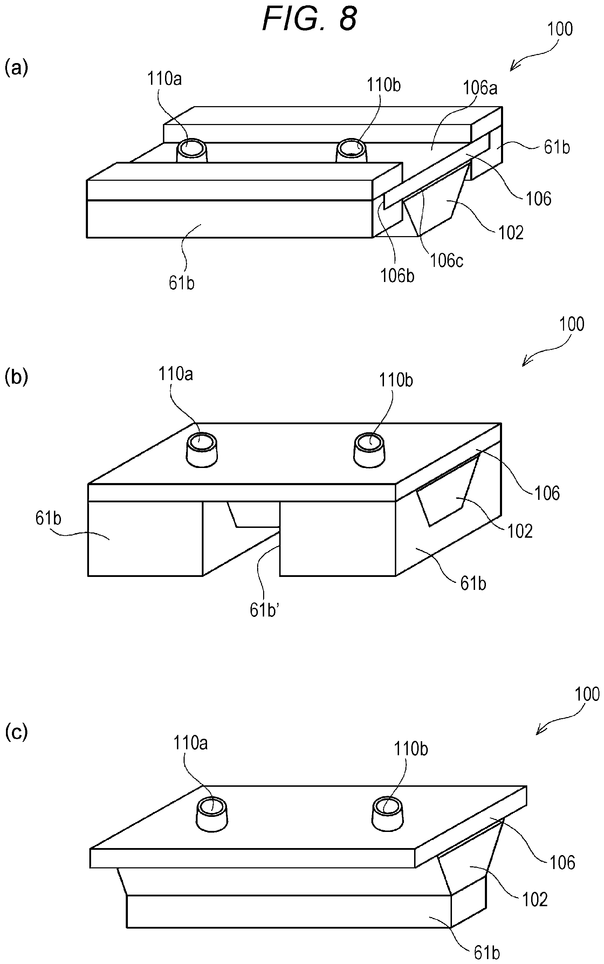

Fig. 8 is a schematic view illustrating a modification of a contact type temperature control unit. The contact type temperature control unit 60 illustrated in Fig. 8 basically has a similar configuration to the contact type temperature control unit 60 illustrated in Fig. 1. Therefore, the same constituent members are denoted by the same reference numerals, and a detailed description thereof will be omitted.

As illustrated in Fig. 8(a), the heat transfer member 61b can also cover an upper surface 106a, a side surface 106b, and a lower surface 106c of the channel forming member 106. In this case, the heat transfer member 61b may be disposed so as to sandwich the upper surface 106a, the side surface 106b, and the lower surface 106c of the channel forming member 106 by a plurality of divided members, or may be disposed so as to be fitted in the channel forming member 106 by an integrally formed member.

As illustrated in Fig. 8(b), the heat transfer member 61b can cover the dielectric member 102. In this case, the heat transfer member 61b has a hole 61b' at a portion in contact with the incident surface 102a and the emitting surface 102c of the dielectric member 102 so as not to interfere with an optical path of excitation light α.

As illustrated in Fig. 8(c), the heat transfer member 61b can be in contact with only the lower surface 102d of the dielectric member 102.

Although not illustrated, the temperature control element 61a can be in contact with the lower surface 102d of the dielectric member 102 without using the heat transfer member 61b.

(Modification of non-contact type temperature control unit)

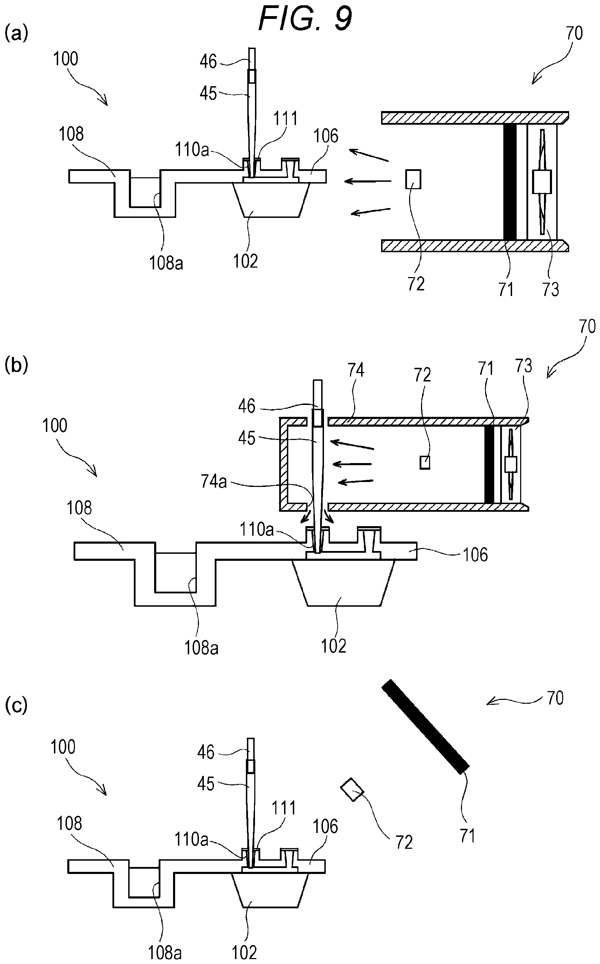

Fig. 9 is a schematic view illustrating a modification of a non-contact type temperature control unit. The non-contact type temperature control unit 70 illustrated in Fig. 9 basically has a similar configuration to the non-contact type temperature control unit 70 illustrated in Fig. 1. Therefore, the same constituent members are denoted by the same reference numerals, and a detailed description thereof will be omitted.

As illustrated in Fig. 9(a), the non-contact type temperature control unit 70 can also be disposed so as to blow heated or cooled air on the sensor chip 100 from the horizontal direction. As described above, if the non-contact type temperature control unit 70 is disposed so as not to interfere with the excitation light irradiation unit 20, the fluorescence detecting unit 30, the transport unit 50, and the like, the non-contact type temperature control unit 70 may blow heated or cooled air on the sensor chip 100 from any direction.

As illustrated in Fig. 9(b), the non-contact type temperature control unit 70 can also be disposed such that heated or cooled air is blown on the pipette tip 45, and the heated or cooled air also hits the sensor chip 100.

In this case, as illustrated in Fig. 9(b), the non-contact type temperature control unit 70 further includes a pipette tip housing 74. The pipette tip housing 74 has a pipette tip hole 74a through which the pipette tip 45 passes. The pipette tip 45 is inserted into the first through hole 110a of the sensor chip 100 in a state of passing through the pipette tip hole 74a.

By blowing heated or cooled air from the non-contact type temperature control unit 70 in this state, the pipette tip 45 and a liquid collected in the pipette tip 45 can be subjected to temperature control. Therefore, temperature control of a liquid introduced into the channel 112 of the sensor chip 100 can be performed in advance, or a liquid in the pipette tip 45 can be subjected to temperature control during reciprocation. A change in temperature of the reaction field caused by the introduction of the liquid into the channel 112 can be suppressed.

The air blown on the pipette tip 45 blows out from a gap between the pipette tip 45 and the pipette tip hole 74a and heats or cools the sensor chip 100.

As described above, by simultaneously heating or cooling the pipette tip 45, the liquid collected in the pipette tip 45, and the sensor chip 100, the temperature of the reaction field is rapidly brought close to the reaction field target temperature T and is easily maintained at the reaction field target temperature T.

As illustrated in Fig. 9(c), the non-contact type temperature control unit 70 can also be constituted by the second temperature controller 71 including an infrared heater that heats the sensor chip 100 by emitting infrared light and the second temperature sensor 72. In this case, the air blower 73 is unnecessary as the non-contact type temperature control unit 70, and therefore it is not necessary to consider an influence of air.

(Modification of sensor chip)

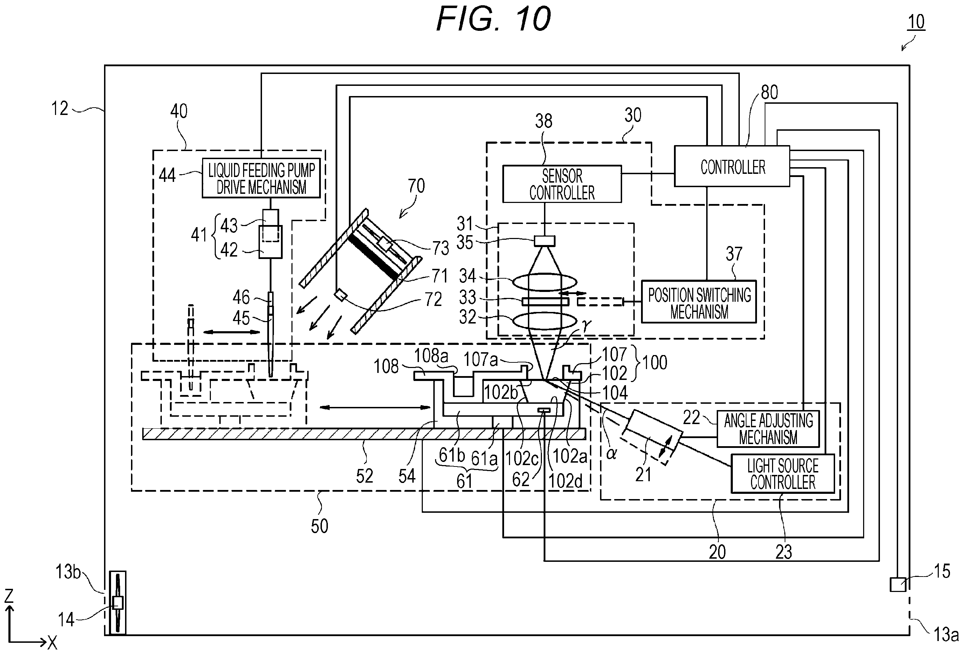

Fig. 10 is a schematic view illustrating a modification of a sensor chip. The sensor chip 100 illustrated in Fig. 10 basically has a similar configuration to the sensor chip 100 illustrated in Fig. 1. Therefore, the same constituent members are denoted by the same reference numerals, and a detailed description thereof will be omitted.

As illustrated in Fig. 10, the liquid holding member of the sensor chip 100 can also serve as a well member 107. The number of wells 107a of the well member 107 may be one as in the present embodiment, or a plurality of the wells 107a may be disposed in a matrix.

In a case where the well member 107 is used in this manner, when heated air is directly blown on a liquid in the well 107a by the non-contact type temperature control unit 70, the liquid is evaporated, and the concentration of a sample liquid or the like may change.

Therefore, preferably, the non-contact type temperature control unit 70 is disposed such that air does not directly hit the liquid in the well 107a, or an upper surface opening of the well 107a is protected by a multilayer film or the like, and the pipette tip 45 introduces a collected liquid into the well 107a in a state of passing through the multilayer film.

The preferable embodiment of the present invention has been described above, but the present invention is not limited thereto. For example, although the SPFS apparatus has been described in the above Examples, the sample detecting system according to the present invention can be modified variously without departing from the object of the present invention. For example, the sample detecting system according to the present invention is applicable to a sample detecting system utilizing fluorescence immunoassay (FIA), such as an SPR apparatus, or a sample detecting system utilizing enzyme immunoassay (EIA).

Reference Signs List

| | |
|---|---|
| 10 | SPFS apparatus |
| 12 | Housing |
| 13a | Intake port |
| 13b | Exhaust port |
| 14 | Fan |
| 15 | Third temperature sensor |
| 20 | Excitation light irradiation unit |
| 21 | Light source unit |
| 22 | Angle adjusting mechanism |
| 23 | Light source controller |
| 30 | Fluorescence detecting unit |
| 31 | Light receiving unit |
| 32 | Lens |
| 33 | Optical filter |
| 34 | Lens |
| 35 | Light receiving sensor |
| 37 | Position switching mechanism |
| 38 | Sensor controller |
| 40 | Liquid feeding unit |
| 41 | Syringe pump |
| 42 | Syringe |
| 43 | Plunger |
| 44 | Liquid feeding pump drive mechanism |
| 45 | Pipette tip |
| 46 | Pipette nozzle |
| 50 | Transport unit |
| 52 | Transport stage |
| 54 | Chip holder |
| 60 | Contact type temperature control unit |
| 61 | First temperature controller |
| 61a | Temperature control element |
| 61b | Heat transfer member |
| 61b' | Hole |
| 62 | First temperature sensor |
| 70 | Non-contact type temperature control unit |
| 71 | Second temperature controller |
| 72 | Second temperature sensor |

| 73 | Air blower |
|---|---|
| 74 | Syringe housing |
| 74a | Syringe hole |
| 80 | Controller |
| 100 | Sensor chip |
| 102 | Dielectric member |
| 102a | Incident surface |
| 102b | Film forming surface |
| 102c | Emitting surface |
| 102d | Lower surface |
| 104 | Metal film |
| 106 | Channel forming member |
| 106a | Upper surface |
| 106b | Side surface |
| 106c | Lower surface |
| 107 | Well member |
| 107a | Well |
| 108 | Liquid storage member |
| 108a | Well |
| 110 | Channel groove |
| 110a | First through hole |
| 110b | Second through hole |
| 111 | Multilayer film |
| 112 | Channel |

**Claims**

1. A sample detecting system that detects an analyte using a sensor chip internally having a reaction field that captures the analyte,
the sample detecting system comprising:

   a contact type temperature control unit disposed in contact with the sensor chip; and
   a non-contact type temperature control unit disposed in non-contact with the sensor chip, wherein
   the contact type temperature control unit includes a first temperature controller and a first temperature sensor that measures a temperature between the first temperature controller and the sensor chip, and performs feedback control of the first temperature controller on a basis of an output value of the first temperature sensor and a predetermined first temperature controller target temperature, and
   the non-contact type temperature control unit includes a second temperature controller and a second temperature sensor that measures a temperature between the second temperature controller and the sensor chip, and performs feedback control of the second temperature controller on a basis of an output value of the second temperature sensor and a predetermined second temperature controller target temperature.

2. The sample detecting system according to claim 1, further comprising a third temperature sensor that measures a temperature of an environment in which the sample detecting system is disposed, wherein
the second temperature controller target temperature is set on a basis of an output value of the third temperature sensor.

3. The sample detecting system according to claim 2, wherein a temperature t3 measured by the third temperature sensor and the second temperature controller target temperature T2 are set so as to satisfy the following formula (1):
[Numerical formula 1]

$$T2 = a \times (t3)^2 + b \times t3 + c \qquad (1)$$

wherein a, b, and c each represent a predetermined coefficient.

4. The sample detecting system according to any one of claims 1 to 3, wherein a difference |T - T1| between a target temperature T of the reaction field and the first temperature controller target temperature T1 and a difference |T - T2| between the target temperature T of the reaction field and the second temperature controller target temperature T2 are set so as to satisfy the following formula (2):

[Numerical formula 2]

$$|T - T1| \leq |T - T2| \tag{2}$$

5. The sample detecting system according to any one of claims 1 to 4, wherein
   the sample detecting system executes a sample detection procedure including a plurality of steps, and
   the second temperature controller target temperature T2 is changeable in each of the steps.

6. The sample detecting system according to claim 5, wherein the second temperature controller target temperature T2 is set such that the difference |T - T2| between the target temperature T of the reaction field and the second temperature controller target temperature T2 is larger as a liquid is introduced into the sensor chip in a shorter time.

7. The sample detecting system according to any one of claims 1 to 6, further comprising a liquid feeding unit that introduces a liquid into the sensor chip, wherein
   the liquid feeding unit includes at least a nozzle and a syringe pump that collect the liquid and supply the liquid to the sensor chip, and
   the non-contact type temperature control unit performs heating or cooling of the nozzle that sucks the liquid therein or discharges the liquid therefrom.

8. The sample detecting system according to any one of claims 1 to 7, wherein
   the sensor chip includes:

   a dielectric member,
   a metal film adjacent to an upper surface of the dielectric member;
   a reaction field adjacent to an upper surface of the metal film; and
   a liquid holding member disposed on an upper surface of the reaction layer, and
   the sample detecting system includes:

   an excitation light irradiation unit that irradiates the metal film with excitation light through the dielectric member; and
   a fluorescence detecting unit that detects fluorescence generated from the fluorescently labeled analyte captured by the reaction field on a basis of excitation light with which the metal film is irradiated.

*FIG. 1*

# FIG. 2

START

MOUNT SENSOR CHIP — S100

MOVE SENSOR CHIP TO LIQUID FEEDING POSITION — S110

START TEMPERATURE CONTROL — S120

CLEAN CHANNEL — S130

MOVE SENSOR CHIP TO MEASUREMENT POSITION — S140

DETECT ENHANCEMENT ANGLE — S150

MEASURE OPTICAL BLANK VALUE — S160

MOVE SENSOR CHIP TO LIQUID FEEDING POSITION — S170

PRIMARY REACTION — S180

CLEAN CHANNEL — S190

SECONDARY REACTION — S200

CLEAN CHANNEL — S210

MOVE SENSOR CHIP TO MEASUREMENT POSITION — S220

DETECT FLUORESCENCE $\gamma$ — S230

END

## FIG. 3

CLEANING
ENHANCEMENT ANGLE DETECTION
OPTICAL BLANK VALUE MEASUREMENT
PRIMARY REACTION
CLEANING
SECONDARY REACTION
CLEANING
DETECTION OF FLUORESCENCE γ

## FIG. 4

REACTION FIELD TEMPERATURE (°C)

ELAPSED TIME (SECOND)

REGION 1    REGION 2    REGION 3

EXAMPLE 1-1
EXAMPLE 1-2
EXAMPLE 1-3

## FIG. 5

## FIG. 6

# FIG. 7

# FIG. 8

(a)

(b)

(c)

# FIG. 9

(a)

(b)

(c)

# FIG. 10

EP 3 598 129 A1

## INTERNATIONAL SEARCH REPORT

| International application No. |
| --- |
| PCT/JP2018/005166 |

**A. CLASSIFICATION OF SUBJECT MATTER**
Int.Cl. G01N33/543(2006.01)i, G01N21/64(2006.01)i, G01N35/00(2006.01)i

According to International Patent Classification (IPC) or to both national classification and IPC

**B. FIELDS SEARCHED**

Minimum documentation searched (classification system followed by classification symbols)
Int.Cl. G01N33/543, G01N21/64, G01N35/00

Documentation searched other than minimum documentation to the extent that such documents are included in the fields searched

| | |
| --- | --- |
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| Y | WO 2015/064757 A1 (KONICA MINOLTA, INC.) 07 May 2015, paragraphs [0006]-[0047], [0060]-[0087], all drawings<br>& US 2016/0266111 A1, paragraphs [0010]-[0061], [0076]-[0149], all figures & EP 3064928 A1 | 1-2, 5-8 |
| Y | JP 2012-215473 A (FUJIFILM CORPORATION) 08 November 2012, paragraphs [0029]-[0057], fig. 1-5<br>& WO 2012/132312 A1 | 1-2, 5-8 |

☒ Further documents are listed in the continuation of Box C.   ☐ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 27.04.2018 | 15.05.2018 |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
| --- | --- |

Form PCT/ISA/210 (second sheet) (January 2015)

**INTERNATIONAL SEARCH REPORT**

International application No.

PCT/JP2018/005166

C (Continuation).    DOCUMENTS CONSIDERED TO BE RELEVANT

| Category* | Citation of document, with indication, where appropriate, of the relevant passages | Relevant to claim No. |
|---|---|---|
| Y | WO 2014/103744 A1 (HITACHI HIGH TECH CORPORATION) 03 July 2014, paragraphs [0015]-[0047], all drawings<br>& JP 6072832 B2 & US 2015/0316532 A1, paragraphs [0028]-[0112], all figures & EP 2940477 A1 & CN 104871007 A | 1-2, 5-8 |
| Y | JP 2009-002801 A (OLYMPUS OPTICAL CO.) 08 January 2009, paragraphs [0032]-[0076], all drawings (Family: none) | 1-2, 5-8 |
| Y | WO 2012/070557 A1 (HITACHI HIGH TECH CORPORATION) 31 May 2012, paragraphs [0013]-[0031], all drawings<br>& JP 5480399 B2 & US 2013/0243652 A1, paragraphs [0017]-[0116], all figures & EP 2645108 A1 & CN 103238073 A | 2 |
| Y | JP 2006-226780 A (FUJIFILM CORPORATION) 31 August 2006, paragraphs [0021]-[0085], all drawings (Family: none) | 2 |
| A | WO 2003/093835 A1 (ARKRAY INC.) 13 November 2003 & US 2005/0164401 A1 & EP 1500936 A1 & CN 1650172 A | 1-8 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012215465 A